# DEMANDE DE BREVET EUROPEEN

(11) **EP 4 501 274 A2**
(43) Date de publication de la demande: **05.02.2025**
(21) Numéro de dépôt: 24220977.3
(22) Date de dépôt: 24.10.2016
(51) Int. Cl.: A61C 9/00

(54) **ELÉMENT DE CICATRISATION POUR UNE RESTAURATION DENTAIRE**

(30) Priorité: 27.10.2015 FR 1560235
(62) Demande divisionnaire de: 22192558.9
(71) Demandeur: Euroteknika, 74700 Sallanches (FR)
(72) Inventeur: LEGER, Théo, 74700 Domancy (FR); CALVAT, Benjamin, 74300 Ugine (FR); LANCIEUX, Cédric, 74120 Megève (FR)
(74) Mandataire: Novaimo

(57) **Abrégé**

Elément de cicatrisation unitaire, adapté à une fixation directe sur un implant dentaire (60) sans utilisation d'une embase de pilier, comprenant une surface latérale (13) destinée à une intégration au sein d'une gencive pour mettre en forme, selon une forme anatomique, la gencive lors de sa cicatrisation, et une surface terminale (14), caractérisé en ce qu'une partie de la surface latérale (13) et de la surface terminale (14) forment une surface émergente, qui est asymétrique par rapport à au moins un plan médian perpendiculaire à la surface émergente et passant par le centre de la surface émergente ou comprenant un axe (18) central de l'élément de cicatrisation et en ce que la surface émergente comprend au moins deux marqueurs informatifs (70) permettant l'identification d'au moins deux caractéristiques dudit élément de cicatrisation et/ou dudit implant dentaire (60) sur lequel il serait connecté.

## Description

La présente invention se rapporte à un élément de cicatrisation pour une restauration dentaire et à un ensemble de cicatrisation comprenant un tel élément de cicatrisation. Elle concerne aussi une méthode de fabrication d'un pilier de restauration dentaire et/ou d'une prothèse basée sur un tel élément de cicatrisation.

La restauration dentaire permet de réaliser une dentition artificielle à un patient partiellement ou totalement édenté. Elle repose sur l'intégration d'un ou plusieurs implants dans la structure osseuse, pratiquée par une incision de la gencive afin d'atteindre la structure osseuse et de la percer. Ensuite, un élément de cicatrisation est en général fixé sur un implant et cet ensemble reste intouché jusqu'à solidarisation de l'implant dans la structure osseuse par ostéointégration et cicatrisation de la gencive autour de l'élément de cicatrisation. La restauration dentaire peut être finalisée par la fixation d'un pilier de restauration sur l'implant, sur lequel est fixée la prothèse dentaire, ou en variante la fixation d'une prothèse dentaire directement sur l'implant. Le pilier et la prothèse dentaire sont personnalisés, adaptés à l'anatomie du patient et à la dent à remplacer, pour atteindre un résultat aussi proche que possible de la dentition naturelle idéale. Pour cela, le volume précis de l'espace à restaurer est en général pris en compte, par une prise d'empreinte, qui permet la fabrication personnalisée de la prothèse dentaire.

Dans l'état de la technique, les méthodes existantes de restauration dentaire se heurtent à tout ou partie des problèmes techniques suivants :
- dans de nombreux procédés existants, une nouvelle intervention sur la gencive est réalisée après sa cicatrisation suite à la pause d'implant, pour réaliser l'empreinte, matérielle ou numérique, de l'espace à restaurer, tout en ayant une vue de l'implant et de la gencive pour prendre en compte précisément l'ensemble de cette géométrie, dans le but de fabriquer un pilier et une prothèse de formes précises : cette approche est naturellement traumatisante ;
- d'autres procédés existants limitent ce traumatisme en utilisant des composants de cicatrisation qui ne sont pas retirés lors d'une prise d'empreinte, pour ne pas heurter la gencive : en contrepartie, ces procédés utilisent des éléments cicatrisants particuliers, en général de forme cylindrique et standard et intégrant parfois des composants complémentaires pour permettre la prise en compte de tout ou partie de la géométrie au-dessus de l'implant sans y avoir totalement accès par une prise d'empreinte. Ces méthodes moins traumatisantes présentent alors d'autres inconvénients, de complexité et/ou de moins bonne optimisation de la phase de cicatrisation.

Ainsi, un objet général de l'invention consiste en une solution de restauration dentaire qui ne comprend pas tout ou partie des inconvénients de l'état de la technique.

Plus précisément, un premier objet de l'invention est une solution de restauration dentaire qui minimise le traumatisme du patient lors du procédé de restauration.

Un second objet de l'invention est une solution de restauration dentaire qui permet une restauration la plus adaptée possible à l'anatomie du patient.

Un troisième objet de l'invention est une solution de restauration dentaire la plus universelle possible, adaptée à tout implant et toute restauration.

Un quatrième objet de l'invention est une solution de restauration dentaire la plus simple possible.

A cet effet, l'invention repose sur un élément de cicatrisation apte à une connexion avec un implant dentaire, comprenant une surface latérale destinée à une intégration au sein d'une gencive pour mettre en forme la gencive lors de sa cicatrisation, et une surface terminale, caractérisé en ce qu'une partie de la surface latérale et de la surface terminale forment une surface émergente, qui est asymétrique par rapport à au moins un plan médian perpendiculaire et en ce que la surface émergente comprend au moins deux marqueurs informatifs permettant l'identification d'au moins deux caractéristiques dudit élément de cicatrisation et/ou d'une embase de pilier et/ou dudit implant dentaire sur lequel il serait connecté.

La surface émergente de l'élément de cicatrisation peut être asymétrique par rapport à au moins un plan médian perpendiculaire à la surface émergente et passant par le centre de la surface émergente ou comprenant un axe central de l'élément de cicatrisation.

Une section transverse à la surface latérale de l'élément de cicatrisation ou une projection sur un plan parallèle à la surface terminale de la surface émergente de l'élément de cicatrisation peut présenter :
- une forme sensiblement trapézoïdale ou une forme sensiblement polygonale, ou triangulaire, ou carrée, ou rectangulaire, ou ovoïde, ou une forme sensiblement polygonale avec des angles arrondis ; et/ou
- une partie destinée à un positionnement orienté vers l'extérieur de la bouche de plus grande dimension qu'une partie destinée à un positionnement orienté vers l'intérieur.

La surface terminale de l'élément de cicatrisation et/ou sa surface émergente peut présenter :
- une surface non plane, courbée ; et/ou
- une surface convexe ; et/ou
- une surface sans aspérité en dehors des marqueurs informatifs ; et/ou
- une surface sans ouverture débouchante.

Lesdites caractéristiques identifiées par les marqueurs informatifs peuvent comprendre un ou plusieurs éléments parmi :
- la hauteur de l'élément de cicatrisation,
- la forme de l'élément de cicatrisation, notamment la forme et les dimensions de la section transverse de sa surface latérale ou de la projection sur un plan parallèle de la surface émergente,
- les dimensions de la partie de liaison de l'élément de cicatrisation avec une embase de pilier, et indirectement d'un l'implant dentaire,
- l'orientation dudit implant dentaire, par l'intermédiaire de l'orientation de l'élément de cicatrisation.

Lesdits au moins deux marqueurs informatifs peuvent comprendre un premier type de marquage informatif d'une première caractéristique de l'élément de cicatrisation et un deuxième type de marquage informatif d'une deuxième caractéristique distincte de l'élément de cicatrisation.

Chaque marqueur informatif peut appartenir à l'un des types suivants :
- un marqueur informatif négatif, notamment en creux dans l'une des surfaces de l'élément de cicatrisation,
- un marqueur informatif positif, notamment en relief sur l'une des surfaces de l'élément de cicatrisation,
- un marqueur informatif de forme particulière et identifiable formée dans l'une des surfaces de l'élément de cicatrisation, notamment de forme polygonale ou de ligne,
- un marqueur informatif constitué par une valeur numérique lisible ou par un code d'identification lisible tel qu'un code barres ou un code datamatrix,
- une puce RFID.

L'invention porte aussi sur une série d'éléments de cicatrisation, caractérisée en ce qu'elle comprend au moins deux éléments de cicatrisation tels que décrits précédemment présentant des formes différentes, ou en ce qu'elle comprend au moins trois éléments de cicatrisation de formes différentes.

Lesdits au moins deux ou au moins trois éléments de cicatrisation peuvent présenter des hauteurs différentes entre eux et/ou présenter des sections transverses de leurs surfaces latérales ou des projections sur un plan parallèle de leurs surfaces terminales différentes entre elles.

L'invention porte aussi sur un ensemble de cicatrisation apte à une connexion avec un implant dentaire lors d'une phase de cicatrisation d'un procédé de restauration dentaire, caractérisé en ce qu'il comprend un élément de cicatrisation tel que décrit précédemment destiné à être entouré au moins partiellement par une gencive et une embase de pilier destinée à une fixation dans un implant, l'embase de pilier comprenant un axe longitudinal sur toute sa longueur apte à un alignement avec l'axe dudit implant.

L'embase de pilier peut présenter une symétrie ou une quasi-symétrie autour de son axe longitudinal.

L'élément de cicatrisation et l'embase de pilier peuvent former deux éléments distincts et assemblés de manière amovible, notamment par clippage, et
- l'embase de pilier peut comprendre un dispositif de connexion avec un implant et un dispositif de connexion avec l'élément de cicatrisation, ces deux dispositifs de connexion étant agencés autour d'un axe aligné et confondu avec l'axe longitudinal de l'embase de pilier, et
- l'élément de cicatrisation peut être agencé autour d'un axe central aligné avec l'axe longitudinal de l'embase de pilier.

L'élément de cicatrisation peut comprendre un élément anti-rotationnel, notamment une gorge, pour coopérer avec un élément anti-rotationnel, notamment un ergot, de l'embase de pilier, et garantir une fixation de l'élément de cicatrisation à orientation unique, sans rotation autour de l'embase de pilier.

Lesdits au moins deux marqueurs informatifs de l'élément de cicatrisation peuvent comprendre un premier marquage informatif d'une première caractéristique de l'embase de pilier et un deuxième marquage informatif d'une deuxième caractéristique de l'embase de pilier.

L'invention porte aussi sur un procédé de fabrication d'un pilier de restauration dentaire et/ou d'une prothèse, destiné à être fixé sur un implant dentaire sur une première extrémité, caractérisé en ce qu'il comprend les étapes suivantes :
- réalisation d'une empreinte physique ou numérique de l'espace buccal comprenant un élément de cicatrisation tel que décrit précédemment fixé sur l'implant dentaire ou un ensemble de cicatrisation tel que décrit précédemment fixé sur l'implant dentaire,
- détermination automatique et/ou à partir de données saisies sur une interface homme machine par un opérateur du positionnement de l'implant dentaire et de l'espace buccal, à partir de ladite empreinte et des marqueurs informatifs de l'élément de cicatrisation.

L'invention est précisément définie par les revendications.

Ces objets, caractéristiques et avantages de la présente invention seront exposés en détail dans la description suivante d'un mode d'exécution particulier fait à titre non-limitatif en relation avec les figures jointes parmi lesquelles :
Les figures 1 et 2 représentent respectivement des vues en perspective d'une embase de pilier utilisée dans un procédé de restauration selon un mode de réalisation de l'invention.
Les figures 3 et 4 représentent respectivement des vues en perspective d'une phase intermédiaire d'association d'un capuchon sur une embase de pilier dans un procédé de restauration selon le mode de réalisation de l'invention.
Les figures 5 et 6 représentent respectivement des vues en perspective de l'ensemble obtenu après fixation d'un capuchon sur une embase de pilier dans un procédé de restauration selon le mode de réalisation de l'invention.
Les figures 7 et 8 représentent respectivement des mêmes vues en perspective sur lesquelles le capuchon est représenté en transparence pour visualiser l'embase de pilier.
La figure 9 représente les dents inférieures et supérieures en vue de dessus.
La figure 10 représente une vue des dents selon une section justa-gingivale.
La figure 11 représente une section horizontale de la dentition au niveau justa-gingival ainsi que les capuchons correspondants retenus selon le mode de réalisation de l'invention.
Les figures 12a à 12d et 13a à 13d représentent respectivement des vues en perspective de dessus et de dessous d'une série de capuchons selon le mode de réalisation de l'invention.
Les figures 14a à 14d représentent respectivement des vues de côté de la série de capuchons selon le mode de réalisation de l'invention.
Les figures 15a à 15d représentent respectivement des vues de dessus de la série de capuchons selon le mode de réalisation de l'invention.
Les figures 16a à 16d représentent respectivement des vues de dessous de la série de capuchons selon le mode de réalisation de l'invention.
Les figures 17a à 17d et 18a à 18d représentent respectivement des vues en perspective de dessus et de dessous d'une phase intermédiaire d'association de la série de capuchons avec des embases de pilier dans un procédé de restauration selon le mode de réalisation de l'invention.
Les figures 19a à 19d représentent respectivement des vues de côté de la série de capuchons assemblés avec des embases de pilier selon le mode de réalisation de l'invention.
Les figures 20 à 22 représentent des vues en coupe par un plan médian vertical illustrant des étapes du procédé de restauration selon un mode de réalisation de l'invention.
La figure 23 illustre une vue en coupe d'un capuchon selon le mode de réalisation de l'invention disposé sur une embase de pilier fixée dans un implant.
La figure 24 illustre une vue en coupe d'un ensemble de cicatrisation comprenant un capuchon entouré de la gencive et disposé sur une embase de pilier fixée dans un implant selon le mode de réalisation de l'invention.
Les figures 25 et 26 représentent deux vues schématiques d'une gencive en coupe au sein de laquelle est fixé un ensemble de cicatrisation selon le mode de réalisation de l'invention.

Le procédé de restauration selon le mode de réalisation de l'invention comprend donc deux phases, comme explicité précédemment : une première phase dite de cicatrisation durant laquelle un ou plusieurs implant(s) sont intégrés dans la structure osseuse du patient par ostéointégration, et durant laquelle un capuchon de cicatrisation particulier associé à une embase de pilier sont utilisés, comme cela va être détaillé par la suite, puis une seconde phase de restauration en tant que telle, durant laquelle une prothèse définitive est mise en place sur le ou les implants par l'intermédiaire d'un pilier de restauration.

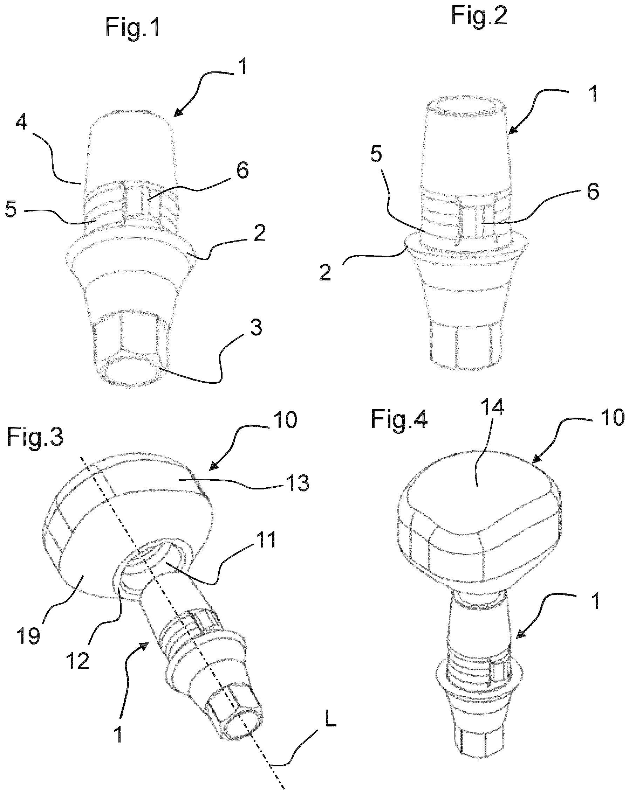

Selon le mode de réalisation de l'invention qui va être décrit, une restauration dentaire utilise, dans la première phase de cicatrisation, un composant intermédiaire que nous appellerons embase de pilier 1, qui est parfois appelé simplement pilier ou T-base ou Esthétibase. L'embase de pilier 1, particulièrement représentée sur les figures 1 à 8, comprend deux parties principales, séparées par une collerette 2. Une première partie comprend un dispositif de connexion 3 avec un implant. Une seconde partie coronaire est destinée à recevoir un capuchon de cicatrisation 10. Pour cela, elle comprend un dispositif de connexion 4 avec un tel capuchon. Selon le mode de réalisation, ce dispositif de connexion 4 comprend un élément de clippage 5 et un élément anti-rotationnel 6, pour empêcher le capuchon de tourner autour de l'axe longitudinal L de l'embase de pilier 1, cet axe longitudinal L étant par ailleurs destiné à un alignement avec l'axe d'un implant. Selon le mode de réalisation, l'élément anti-rotationnel 6 est un ergot. Cet élément anti-rotationnel 6 est de plus aligné avec une surface particulière du dispositif de connexion 3 avec un implant. De plus, selon le mode de réalisation, l'élément de clippage 5 est formé par plusieurs gorges agencées sur la circonférence de l'embase de pilier 1 au voisinage de la collerette 2. De manière avantageuse, l'élément de clippage 5 est tel qu'il permet d'obtenir un clic sonore lors de la fixation par clippage d'un capuchon de cicatrisation 10.

En remarque, l'embase de pilier 1 utilisée est non définitive, participe à la première phase de cicatrisation uniquement, est de préférence retirée lors de la finalisation de la restauration et remplacée par un pilier de restauration définitif (qui peut se présenter sous la forme d'une autre embase). En variante, la même embase de pilier 1 est éventuellement retirée, nettoyée et réutilisée dans la restauration définitive, remplissant alors la seconde fonction de pilier de restauration.

De plus, l'embase de pilier 1 est de préférence universelle, et présente une forme symétrique ou plus précisément quasi symétrique (l'élément anti-rotationnel forme par exemple une exception à la symétrie) autour d'un axe longitudinal L qui forme un axe de révolution. Cet axe L forme donc notamment un axe central de symétrie du dispositif de connexion 3 avec un implant. Avantageusement, ce même axe L forme aussi un axe de symétrie du dispositif de connexion 4 avec un capuchon. L'embase de pilier 1 s'étend donc globalement selon une seule direction, identifiée par un axe longitudinal L unique. Nous pouvons donc plus généralement considérer que l'embase de pilier comprend un axe longitudinal L qui s'étend sur toute sa longueur, de manière apte à un alignement avec l'axe d'un implant 60. Notamment, les deux dispositifs de connexion 3, 4 de l'embase de pilier 1 sont agencés à des niveaux différents autour de ce même axe longitudinal L.

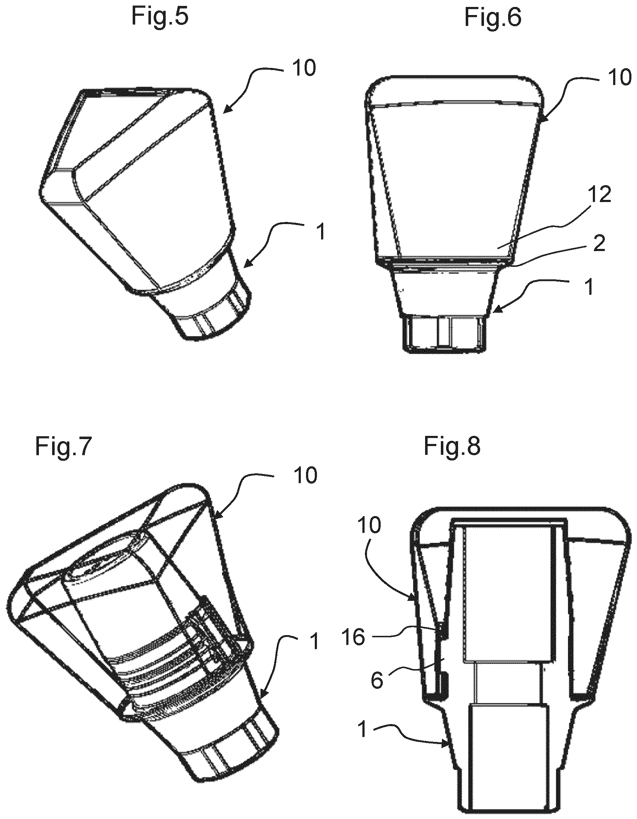

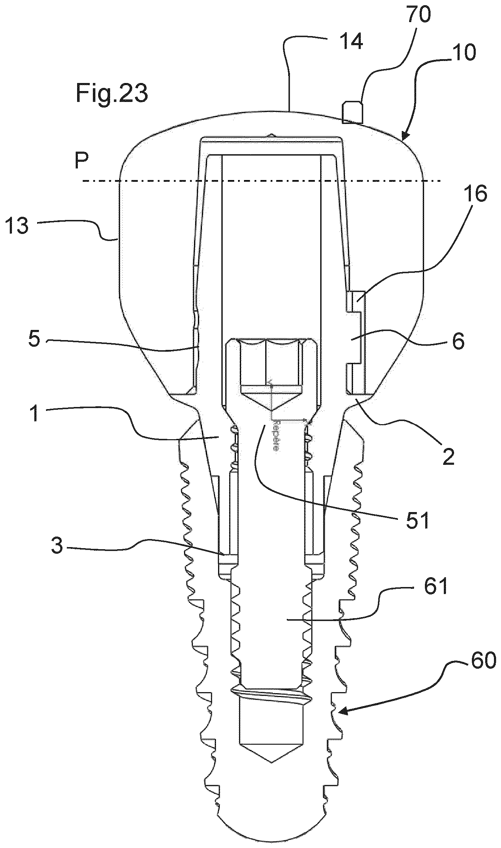

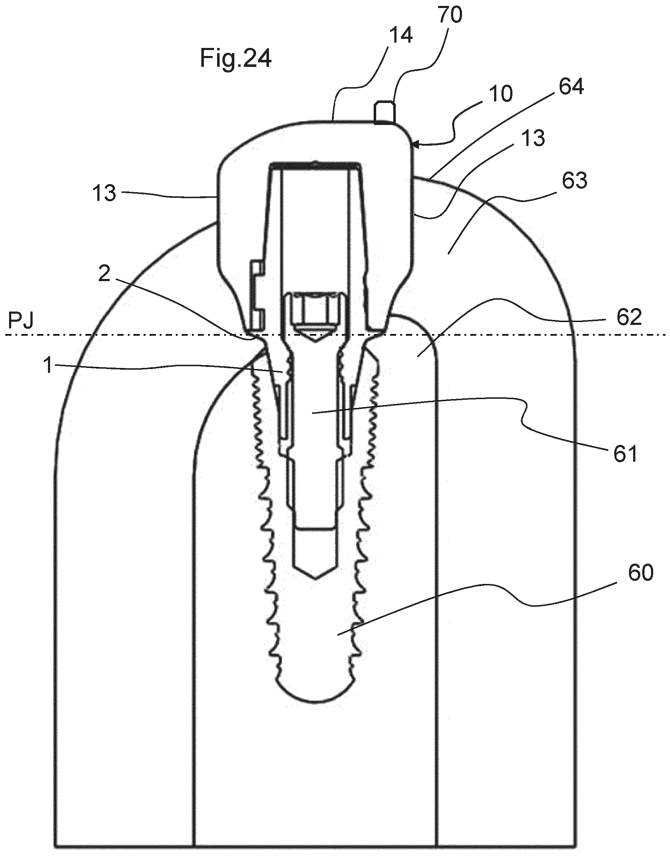

Suite à la fixation d'un implant 60 lors d'un procédé de restauration dentaire, une embase de pilier 1 est fixée sur l'implant 60 par son dispositif de connexion 3, et par l'intermédiaire d'une vis 61, puis un capuchon 10 est fixé sur la seconde partie coronaire de l'embase de pilier. Cet assemblage est illustré par les figures 23 et 24. Comme illustré notamment par les figures 3 et 7, le capuchon 10 comprend une ouverture 11 pour former une partie de liaison et un volume intérieur creux, destiné à l'insertion de la seconde partie coronaire de l'embase de pilier. Le pourtour de cette ouverture 11 comprend une surface 12 destinée à venir en appui sur une surface plane correspondante de la collerette 2 de l'embase de pilier, après clippage du capuchon 10 sur l'embase de pilier, pour atteindre l'ensemble assemblé représenté par les figures 5 à 8 et 19a à 19d.

Il existe autant d'embases de pilier différentes que de dispositifs de connexion différents des implants existants, afin de pouvoir disposer, pour chaque implant existant, d'une embase de pilier dotée d'un dispositif de connexion 3 qui lui est adapté. L'avantage de cette approche est qu'elle permet de conserver toute la seconde partie coronaire des embases de pilier, à partir de la collerette 2, inchangée, quel que soit l'implant correspondant à l'embase de pilier. Naturellement, il est aussi possible de prévoir des secondes parties coronaires différentes pour des embases de pilier différentes, selon leur utilisation envisagée. Dans tous les cas, la seconde partie coronaire de l'embase de pilier est indépendante de l'implant, décorrélée du dispositif de fixation de l'implant.

Le capuchon 10 a pour fonction de venir se loger au sein de la gencive incisée, après fixation d'un implant, par fixation, de préférence amovible, sur une embase de pilier connectée à l'implant. La configuration finale est représentée sur la figure 24. Dans cette configuration, l'implant 60 est solidarisé à la partie osseuse 62, l'embase de pilier 1 est fixée sur l'implant 60, de sorte que sa collerette 2 se trouve positionnée au niveau de la frontière entre la partie osseuse 62 et la gencive 63. Le capuchon 10 recouvre l'embase de pilier 1 jusqu'à la collerette 2, de sorte que la gencive 63 est presque exclusivement en contact avec le capuchon 10. L'ensemble formé par l'assemblage d'un capuchon sur une embase de piler correspond ainsi à un ensemble de cicatrisation, qui participe temporairement au procédé de restauration, permettant la cicatrisation et la fabrication sans heurt de la prothèse définitive, comme cela sera détaillé par la suite.

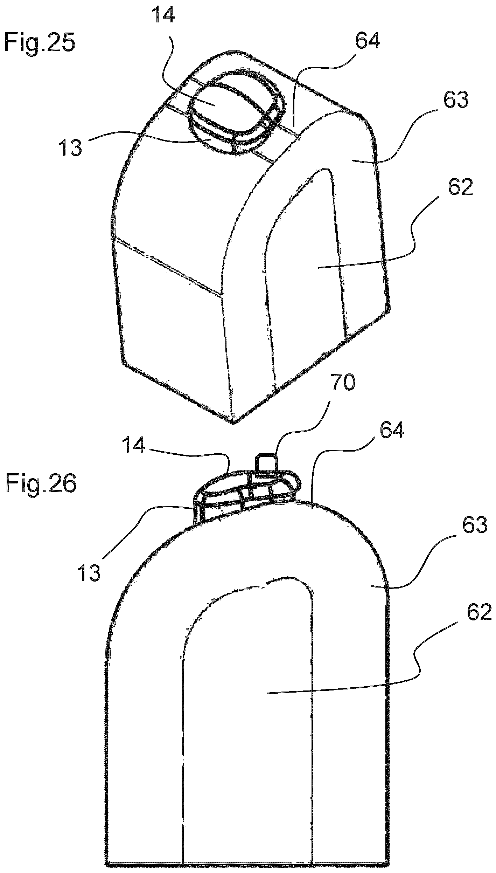

La gencive 63 se cicatrise donc autour de la surface latérale 13 du capuchon 10. Pour cela, cette surface latérale 13 est choisie pour correspondre au mieux au milieu buccal du patient. La surface terminale 14 opposée à l'ouverture 11 du capuchon est destinée à rester visible au-dessus de la surface gingivale 64 de la gencive 63, ou au moins partiellement visible, puisque la gencive reste principalement en contact avec la surface latérale 13 du capuchon. En remarque, au moins une partie de la surface terminale 14 et éventuellement une partie haute de la surface latérale 13 forment donc une surface émergente du capuchon. Cette surface émergente est notamment illustrée par les figures 25 et 26. Pour cela, des capuchons de hauteur différente peuvent être prévus pour s'adapter à différentes configurations de la géométrie buccale. A titre d'exemples de réalisation, trois hauteurs différentes standard permettent une bonne adaptation à toutes les situations. Cette hauteur est avantageusement comprise entre 3 et 7 mm. Du fait de l'utilisation d'une embase de pilier qui joue le rôle d'interface, un même capuchon 10 est ainsi universellement adapté à tous les implants existants.

Selon le mode de réalisation de l'invention, la forme du capuchon est spécifiquement choisie pour favoriser la cicatrisation de la gencive, selon une forme anatomique correspondant au mieux à la dent à remplacer et par conséquent aussi à la future prothèse destinée à occuper cet espace buccal. Cette forme est notamment caractérisée par la section plane de sa surface latérale 13, cette section étant une section transverse par un plan perpendiculaire P à la surface latérale 13, représenté sur la figure 23, et sensiblement parallèle à la surface terminale 14. En remarque, cette section est sensiblement reproduite par la forme de la surface terminale 14, ou plus précisément par la projection de cette surface terminale 14 sur un tel plan perpendiculaire, c'est-à-dire sensiblement parallèle à la surface gingivale 64.

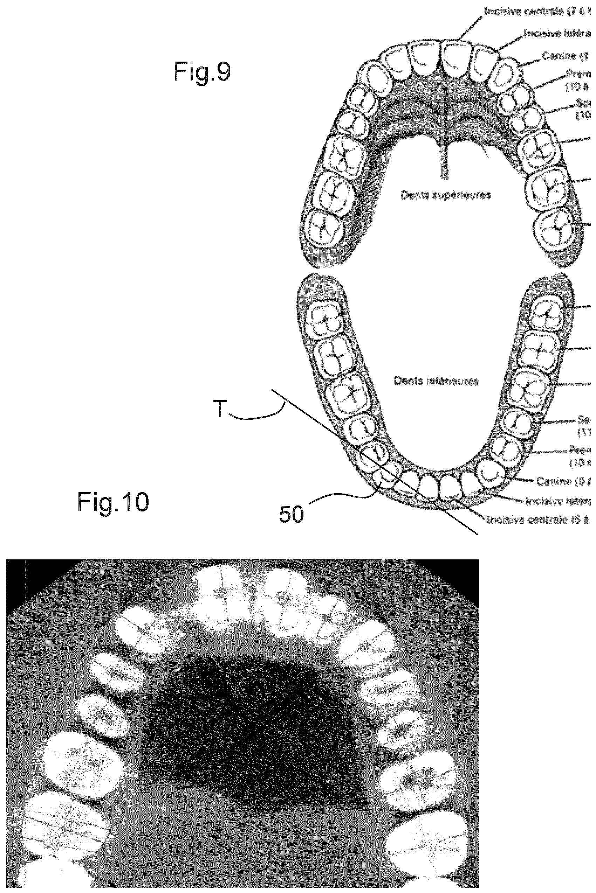

Pour comprendre l'approche retenue, la figure 9 illustre une vue de dessus des dents supérieures et inférieures et la figure 10 illustre une vue en coupe au niveau du plan justogingival PJ d'une dentition, représenté sur la figure 24, au niveau de la racine des émergences des dents. Ces figures montrent que les dents possèdent des sections de formes différentes, que l'on peut simplifier par des formes rectangulaires et/ou carrées et/ou triangulaires, mais plus précisément trapézoïdales.

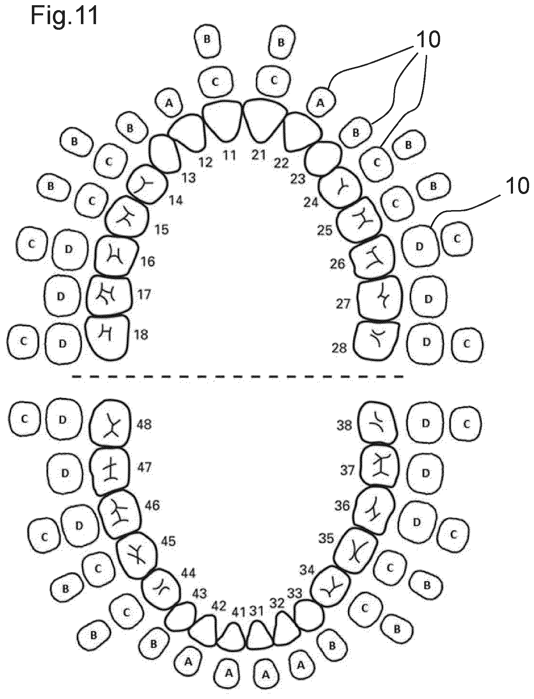

Selon le mode de réalisation choisi, une série de capuchons 10 de formes différentes va permettre de reproduire au mieux ces différentes formes. La figure 11 représente ainsi une vue de dessus des sections de toutes les dents et une vue de dessus de capuchons 10 associés à chaque dent. Les formes des différentes séries de dents, numérotées de 11 à 18, de 21 à 28, de 31 à 38 et de 41 à 48 sur cette figure, ces numéros ne devant pas être confondus avec les références numériques utilisées par ailleurs sur les autres figures pour désigner les caractéristiques de l'invention, sont toutes approchées par l'intermédiaire de quatre capuchons 10 différents, référencés A à D. Pour certaines dents, voire toutes les dents, plusieurs capuchons, parmi les capuchons A à D, apparaissent adaptés.

Comme il le sera détaillé plus loin, un élément de cicatrisation objet de l'invention pourra indifféremment correspondre soit à l'ensemble formé par une embase de pilier et par l'un des capuchons de la série fixé sur l'embase de pilier à l'opposé de sa liaison à l'implant dentaire, soit à un élément de cicatrisation unitaire directement fixé sur l'implant dentaire 60 sans utilisation de l'embase de pilier.

Ainsi de manière plus générale, une série d'éléments de cicatrisation sera telle que les éléments de cicatrisation qui la composent présentent des hauteurs différentes entre eux et/ou présentent des sections transverses de leur surfaces latérales 13 ou des projections sur un plan parallèle de leurs surfaces émergentes ou terminales différentes entre eux.

Au sein d'une même série d'éléments de cicatrisation, les sections transverses des surfaces latérales 13 ou les projections sur un plan parallèle de leurs surfaces émergentes ou terminales 14 sont chacune parmi : une forme sensiblement trapézoïdale ou une forme sensiblement polygonale, ou triangulaire, ou carrée, ou rectangulaire, ou ovoïde, ou une forme sensiblement polygonale avec des angles arrondis, ou cylindrique.

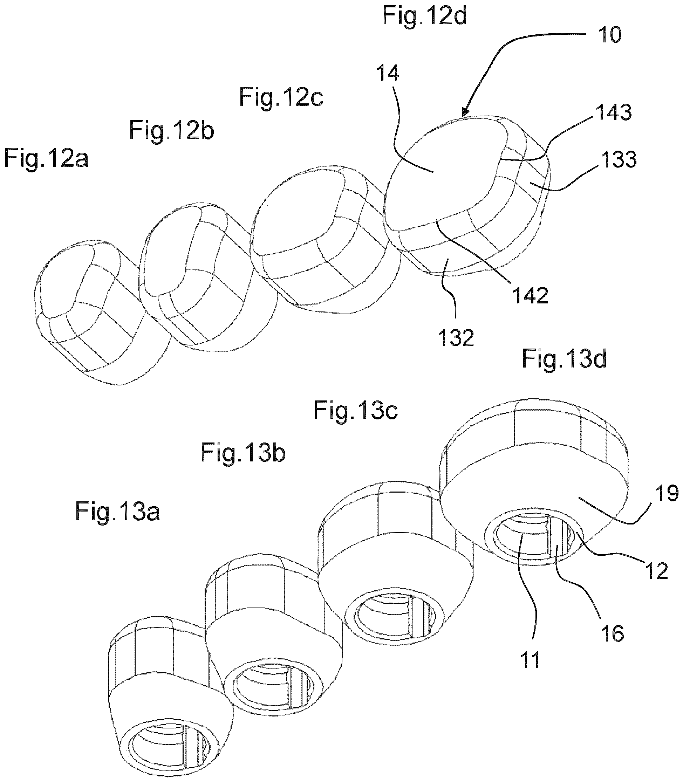

Dans l'exemple de réalisation choisi et illustré par la figure 11, les capuchons A sont adaptés pour traiter la restauration des incisives latérales supérieures et toutes les incisives inférieures. Les capuchons B sont adaptés pour la restauration des canines et des prémolaires, les capuchons C sont adaptés pour la restauration des molaires intermédiaires puis les capuchons D sont adaptés pour la restauration des molaires les plus grosses. Ces capuchons vont maintenant être décrits plus en détail. Le capuchon A est particulièrement illustré par les figures 12a à 16a, le capuchon B par les figures 12b à 16b, le capuchon C par les figures 12c à 16c et le capuchon D par les figures 12d à 16d. Pour ne pas alourdir les figures, les références numériques ne sont pas reproduites sur tous les capuchons de ces figures ; toutefois, tous ces capuchons possèdent les mêmes caractéristiques, qui vont être décrites.

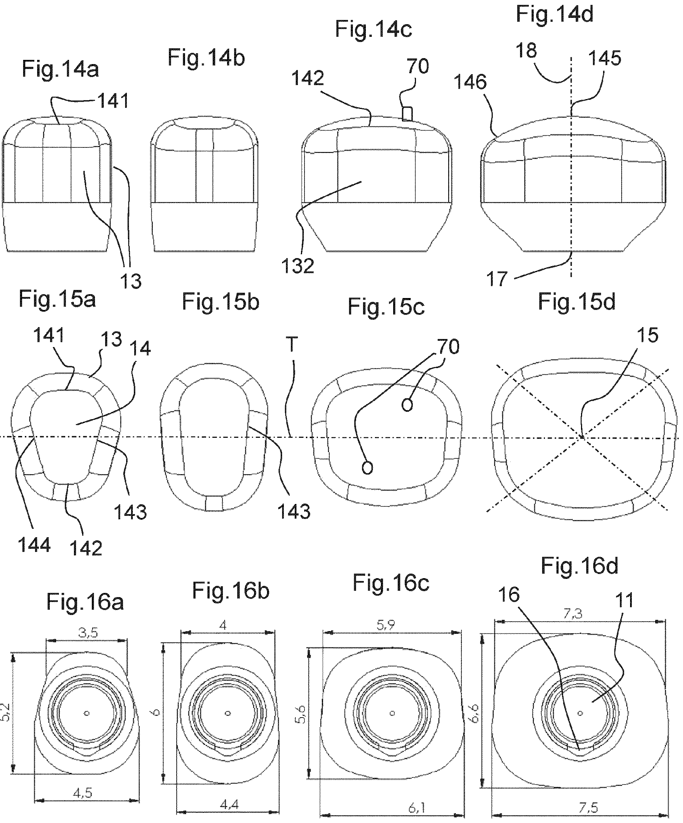

Comme cela ressort des figures 12 et 15, les surfaces terminales 14 de ces capuchons 10 (A à D), destinées à un positionnement au-dessus de l'émergence gingivale, sont sensiblement planes et destinées à un positionnement parallèle à un plan horizontal (parallèle au plan justa-gingival PJ, entre 1 et 2 mm inclus au-dessus de ce plan) correspondant au plan de coupe de la figure 9. Elles sont toutefois légèrement bombées, présentant une partie centrale 145, plus particulièrement visible sur les figures 14a à 14d, destinée à s'élever plus au-delà de la gencive que ses parties périphériques 146.

La section transverse du capuchon, par un plan P perpendiculaire à sa surface latérale 13, comme explicité précédemment, qui donne la forme finale à la gencive après cicatrisation, est sensiblement reproduite par la surface terminale 14 du capuchon, qui vient dans son prolongement. Les sections de tous les capuchons présentent toutes une forme sensiblement trapézoïdale. Elles comprennent un grand côté 141, qui sera disposé du côté de l'extérieur de la bouche (côté vestibulaire), un petit côté 142 opposé parallèle, qui sera disposé du côté de l'intérieur de la bouche (côté lingual), reliés par deux côtés 143, 144. Le croisement des diagonales du trapèze permet de définir un centre 15. De plus, en considérant le centre 17 de l'ouverture 11 sensiblement circulaire de la surface opposée du capuchon 10, il est possible de définir un axe 18 central du capuchon, passant par les deux points centraux 15, 17. Cet axe 18 du capuchon 10 est perpendiculaire à la surface terminale 14. L'ensemble de l'architecture de l'embase de pilier et d'un capuchon 10 associés est conçu pour que l'axe 18 du capuchon corresponde à l'axe longitudinal L de l'embase de pilier, et à l'axe de l'implant.

Les quatre types de capuchons 10, A, B, C et D, diffèrent donc notamment par la forme trapézoïdale de la section transverse de leurs surfaces latérales 13. Le trapèze du plus petit capuchon A se rapproche d'un triangle, car son petit côté 142 est très réduit. Le trapèze du capuchon B se rapproche d'un rectangle, dont le grand côté va de l'extérieur de la bouche vers l'intérieur, et correspond aux côtés 143, 144. Au contraire, les trapèzes des capuchons C et D se rapprochent d'un rectangle, voire d'un carré, dont le grand côté est dans le sens inverse, et correspond aux côtés 141, 142, qui sont de longueur proches mais légèrement différentes. A titre d'exemples de réalisation, les figures 16a à 16d donnent des ordres de grandeur des dimensions de ces capuchons, en millimètres.

Naturellement, cette forme sensiblement trapézoïdale retenue présente des angles arrondis et des côtés courbés, pour garantir de ne pas heurter la gencive. De plus, la surface terminale 14 de chaque capuchon, en faisant abstraction des marqueurs agencés sur cette surface et qui seront décrits plus loin, présente une surface continue, sans reliefs, et/ou sans partie creuse, et/ou sans gorge, et/ou sans arrête, et/ou sans aspérité. Cette surface est convexe. Notamment, elle ne présente pas de forme creuse, et naturellement pas d'ouverture débouchante (traversante), comme cela serait nécessaire si il était choisi de fixer le capuchon par une vis de fixation. Cette géométrie sans aspérité est favorable à l'hygiène buccale, empêche par exemple l'accumulation d'aliments et le dépôt de la plaque dentaire.

En variante, la série de capuchons pourrait comprendre un nombre différent de géométries différentes, par exemple au moins trois, voire au moins deux. Dans une variante de réalisation simplifiée, une seule forme de capuchon pourrait convenir pour toutes les dents.

Selon d'autres variantes de réalisation, la section transverse d'un capuchon au niveau de sa surface latérale 13 pourrait s'approcher de tout polygone, comme un polygone à trois, cinq ou six côtés. En variante, les angles de ces polygones pourraient être si arrondis que la forme globale s'approcherait d'une forme oblongue, voire de section d'ovoïde, voire de toute autre forme plus éloignée d'un polygone. Avantageusement, cette forme comprend au moins un centre ou point parfaitement défini géométriquement pour définir un centre 15, voire un éventuel axe 18 du capuchon, ce centre étant avantageusement, mais non obligatoirement, dans l'alignement de l'axe longitudinal L de l'embase de pilier.

Selon le mode de réalisation, la géométrie de la surface émergente visible du capuchon du côté de l'intérieur de la bouche diffère de la géométrie du côté de l'extérieur, pour tenir compte de la courbure de la gencive. Cette forme de la surface émergente du capuchon est donc asymétrique par rapport à un plan médian contenant la tangente T de la gencive et passant au centre du capuchon, dit plan tangent médian ; cette tangente T (et donc la projection du plan tangent médian) est représentée sur les figures 15a à 15d et plus précisément sur la figure 9 en considérant une dent 50 à restaurer. Ce plan médian, dit plan tangent T, est parallèle à la tangente T à la gencive, perpendiculaire au plan justogingival PJ, passe au milieu 15 d'un capuchon.

Ainsi, une forme de section circulaire pour la section du capuchon, associée par exemple à un capuchon cylindrique, est inadaptée. Plus généralement, toute section plane présentant une symétrie autour d'un point ou d'un axe est peu ou pas adaptée pour la section susmentionnée du capuchon, car elle ne serait pas adaptée à l'anatomie de la bouche. Pour les mêmes raisons, la surface émergente et visible du capuchon, notamment la surface terminale 14, n'est plus généralement donc pas symétrique par rapport à au moins un, ou même plusieurs plans comprenant son axe 18. Elle n'est pas symétrique par rapport à au moins un, ou plusieurs plans perpendiculaires à la surface terminale 14 et passant par son centre 15. Nous appellerons plan médian perpendiculaire un plan correspondant à une définition donnée ci-dessus. Un tel plan est sensiblement perpendiculaire à la surface terminale 14 de l'élément de cicatrisation, et donc aussi sensiblement perpendiculaire au plan justogingival formant la surface finale de la gencive, d'où émergent les dents. Il passe au centre de la surface terminale 14. Alternativement, un plan médian perpendiculaire peut se définir comme tout plan contenant l'axe 18 du capuchon. Dans l'exemple représenté, particulièrement visible sur les figures 15a à 15d, seul le plan médian perpendiculaire au plan tangent T mentionné, passant au milieu des deux côtés 141, 142, forme un plan de symétrie.

Les surfaces terminales 14 des capuchons sont prolongées à partir de leur périphérie 146 par la surface latérale 13 autour de laquelle principalement se cicatrise la gencive, et qui donne ainsi la forme de gencive adaptée à la future prothèse. Cette surface latérale 13 présente plusieurs surfaces 131, 132, 133, 134, sensiblement planes, éventuellement légèrement courbées, s'étendant selon une direction sensiblement parallèle à l'axe 18 du capuchon et/ou parallèlement à l'axe longitudinal L de l'embase de pilier, en prolongeant respectivement les différents côtés 141, 142, 143, 144 de la surface terminale 14 du capuchon. Les interfaces entre la surface terminale 14 et ces différentes parties de la surface latérale 13 sont réalisées par des surfaces arrondis, sans aspérité, notamment convexes.

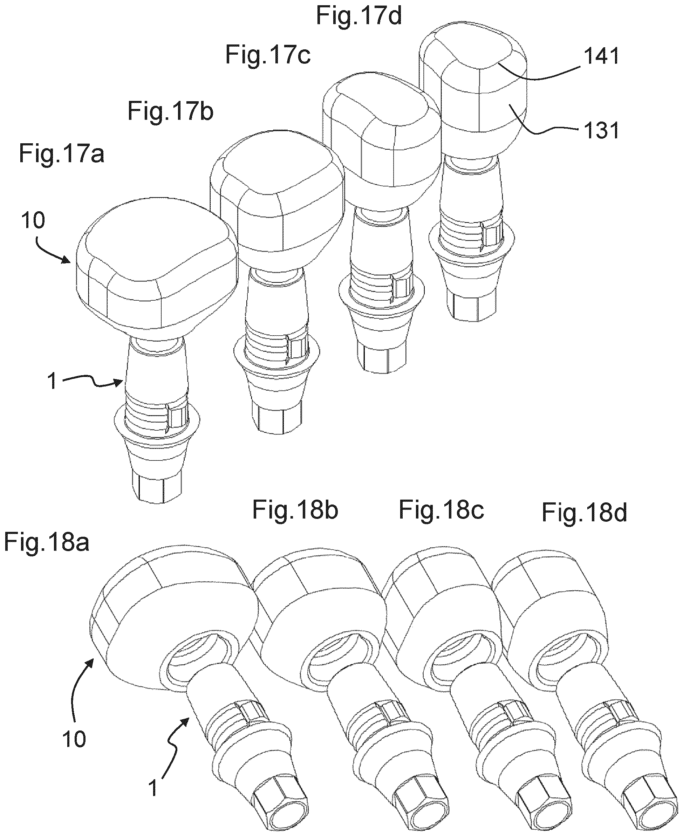

Enfin, la surface latérale 13 des capuchons se termine par une surface 19 sensiblement tronconique, jusqu'à l'ouverture 11 sensiblement circulaire, mentionnée précédemment. Cette ouverture 11 débouche sur une partie creuse interne au capuchon 10, qui permet le logement de la seconde partie coronaire de l'embase de pilier. Cette partie creuse est dotée d'un dispositif de fixation complémentaire à celui 4 de l'embase de pilier. Dans le mode de réalisation, il s'agit de bourrelets prévus pour se clipper sur les gorges 5 de l'embase de pilier. Enfin, une gorge 16 sensiblement longitudinale est agencée dans cette partie creuse du capuchon, pour coopérer avec l'ergot, formant ainsi une liaison bloquée en rotation, et parfaitement indexée, l'orientation du capuchon étant unique et parfaitement imposée. Les figures 3 à 8 et 17a à 17d, 18a à 18d et 19a à 19d, montrent particulièrement un ensemble de cicatrisation selon l'invention, formé par l'assemblage d'un capuchon 10 avec une embase de pilier 1.

Le capuchon peut être formé en matériau plastique, compatible avec une utilisation médicale, et de couleur rose, blanche ou crème. En variante, il peut être en métal, par exemple en titane, ou peut être en zircone. L'utilisation des capuchons de cicatrisation permet donc de favoriser une cicatrisation idéale de la gencive dans un procédé de restauration dentaire, comme cela a été discuté, du fait de sa géométrie conçue en phase avec l'anatomie buccale. La solution a été décrite avec un capuchon de cicatrisation amovible et distinct d'une embase dans le mode de réalisation précédent. En remarque, cet élément de cicatrisation peut en variante être totalement sous-gingival et invisible, puis rendu visible par intervention sur la gencive pour mettre en oeuvre le reste du procédé de reconnaissance qui est décrit ci-après. Dans ce cas, la partie extrême du capuchon sera toujours appelée abusivement partie émergente. Le capuchon 10 sera aussi plus généralement appelé « élément de cicatrisation » 10 par la suite.

Pour chaque capuchon de cicatrisation 10 tel que décrit précédemment, la surface émergente, formée par la surface terminale 14 et éventuellement la partie supérieure de la surface latérale 13, comprend avantageusement au moins deux marqueurs informatifs permettant l'identification d'au moins deux caractéristiques du capuchon de cicatrisation 10 et/ou indirectement de l'embase de pilier 1 et/ou de l'implant dentaire 60. L'aménagement de tels marqueurs informatifs sur le capuchon de cicatrisation 10 permet de ne pas avoir recours à une technique de prise d'empreinte traditionnelle sur l'implant et de réduire le temps requis et la complexité pour mettre au point ensuite le pilier de restauration dentaire qui viendra se placer en remplacement du capuchon de cicatrisation 10 et de l'embase de pilier 1, et sur lequel sera agencée la prothèse finale.

Selon un mode de réalisation, les caractéristiques identifiées par les marqueurs informatifs comprennent un ou plusieurs éléments parmi :
- la hauteur du capuchon de cicatrisation 10,
- la forme du capuchon de cicatrisation 10, notamment la forme et les dimensions de la section transverse de sa surface latérale 13 ou de la projection sur un plan parallèle de la surface émergente,
- les dimensions de la partie de liaison du capuchon de cicatrisation 10 avec l'embase de pilier, et donc indirectement de l'implant dentaire 60,
- l'orientation de l'implant dentaire 60, par l'intermédiaire de l'orientation du capuchon, par exemple par un marqueur aligné avec un élément anti-rotationnel du capuchon, par exemple la gorge 16.

Selon un mode de réalisation particulier, lesdits au moins deux marqueurs informatifs comprennent un premier type de marquage informatif d'une première caractéristique du capuchon de cicatrisation 10 et un deuxième type de marquage informatif d'une deuxième caractéristique du capuchon de cicatrisation 10. Ces première et deuxième caractéristiques liées au capuchon de cicatrisation 10 sont en particulier la hauteur de l'élément de cicatrisation 10 et/ou la forme du capuchon de cicatrisation 10, notamment la forme et les dimensions de la section transverse de sa surface latérale 13 ou de la projection sur un plan parallèle de la surface émergente. Indirectement, un marqueur peut permettre d'en déduire une information sur l'embase de pilier et éventuellement sur l'implant, comme leur orientation, qui peut être liée à l'orientation du capuchon.

Selon que cela ne soit limitatif quant à la liberté de conception des marqueurs informatifs, chaque marqueur informatif appartient notamment à l'un des types suivants :
- un marqueur informatif négatif, notamment en creux dans l'une des surfaces du capuchon de cicatrisation 10, particulièrement sur sa surface émergente,
- un marqueur informatif positif, notamment en relief sur l'une des surfaces du capuchon de cicatrisation 10, particulièrement sur sa surface émergente,
- un marqueur informatif de forme particulière et identifiable formée dans l'une des surfaces du capuchon de cicatrisation 10, notamment de forme polygonale ou de ligne,
- un marqueur informatif constitué par une valeur numérique lisible ou par un code d'identification lisible tel qu'un code barres et/ou un code datamatrix. Ce marqueur peut être composé de chiffres et/ou de lettres et/ou de tout symbole et/ou de couleurs et/ou de marquages laser ;
- un moyen informatif constitué d'une puce RFID.

En remarque, les considérations géométriques décrites précédemment pour caractériser l'élément de cicatrisation sont vraies en faisant abstraction des marqueurs informatifs. Pour cette raison, ces marqueurs 70 ne sont pas illustrés sur les figures, à l'exception des figures 14c, 15c, 23, 24 et 26, pour ne pas les alourdir. Toutefois, ils sont bien présents sur tous les capuchons.

A titre d'exemple, la caractérisation du marqueur informatif négatif, qui est par exemple un simple trou, est par exemple sa position par rapport au reste du capuchon 10 dans lequel il est formé, sa dimension, sa forme ou le nombre de tels marqueurs informatifs négatifs. Ces caractéristiques de chaque marqueur informatif négatif permettent justement de caractériser la caractéristique du capuchon de cicatrisation 10 et/ou de l'embase de pilier 1 caractérisée par le(s) marqueur(s) informatif(s) négatif(s), comme par exemple la hauteur de l'élément de cicatrisation. Il peut par exemple ensuite en être déduit la position en hauteur de l'embase de pilier 1.

Toujours à titre d'exemple, ces mêmes caractéristiques peuvent être déduites de la caractérisation du marqueur informatif positif, qui est par exemple sa position par rapport au reste du capuchon 10 dans lequel il est formé, sa dimension, sa forme ou le nombre de tels marqueurs informatifs positifs. Ces caractéristiques de chaque marqueur informatif positif permettent justement de caractériser la caractéristique du capuchon de cicatrisation 10 et/ou de l'embase de pilier 1 et donc de l'implant dentaire 60 caractérisée par le(s) marqueur(s) informatif(s) positif(s).

Un autre exemple de tels marqueurs informatifs de formes particulières et identifiables est une encoche formée directement dans le capuchon de cicatrisation 10 lui-même. La présence conjointe de deux telles encoches et la distance les séparant peut servir à indiquer par exemple la position et le type d'embase de pilier et par relation l'implant dentaire 60. Un troisième exemple de tels marqueurs informatifs de formes particulières et identifiables est une ligne, telle qu'une gravure ou un relief, solidaire du capuchon de cicatrisation 10. La dimension, la position de cette ligne et/ou le nombre de telles lignes peuvent servir à caractériser une caractéristique supplémentaire du capuchon de cicatrisation 10 et/ou de l'embase de pilier 1 et donc de l'implant dentaire 60.

De manière générale, la fabrication d'un pilier de restauration dentaire, destiné à être fixé sur l'implant dentaire 60 sur une première extrémité et à recevoir une prothèse sur sa seconde extrémité, comprend les étapes suivantes :
- la réalisation d'une empreinte physique ou numérique de l'espace buccal comprenant un élément de cicatrisation tel que décrit précédemment et fixé sur l'implant dentaire 60, par exemple l'un des capuchons 10 de la série,
- la détermination automatique, à partir de cette empreinte et des marqueurs informatifs de l'élément de cicatrisation, de l'espace buccal de l'embase de pilier 1 et donc du positionnement de l'implant dentaire 60. Outre les avantages décrits précédemment, l'élément de cicatrisation permet la mise en oeuvre d'un procédé de restauration avantageux, comprenant un procédé de fabrication d'une prothèse dentaire et d'un pilier définitifs, avec une traumatisation minimale de la gencive. En effet, il est possible d'obtenir une empreinte numérique ou physique de la zone à restaurer sans retirer l'élément de cicatrisation en bouche, donc sans heurter la gencive. Ainsi, en plus de sa première fonction de cicatrisation, détaillée précédemment, le capuchon remplit une seconde fonction lors du procédé de restauration, en permettant la définition avantageuse de la forme du pilier de restauration et/ou de la prothèse avant son retrait. Cette fonction est complémentaire de sa première fonction de cicatrisation puisqu'elle permet de ne pas traumatiser la gencive après sa cicatrisation selon une forme anatomique avantageuse choisie.

Pour cela, en fin de la phase de cicatrisation du procédé de restauration dentaire, un praticien peut prendre une empreinte numérique de la bouche du patient, sans retirer l'élément de cicatrisation (le capuchon). Les données de numérisation, obtenues par tout appareil comme un scanner buccal par exemple, sont transmises automatiquement à un ordinateur doté d'un logiciel de restauration dentaire. Ce logiciel est doté d'une interface homme machine, par laquelle un opérateur peut indiquer le modèle de capuchon qu'il a utilisé, ou plus généralement la référence de l'élément de cicatrisation, et éventuellement de l'implant et/ou de l'embase de pilier utilisé. Les marqueurs informatifs peuvent être indiqués au logiciel par l'opérateur lui-même ou identifiés automatiquement par le logiciel par tout moyen de détection, de reconnaissance et d'identification adapté en fonction de la nature du marqueur et de sa caractérisation.

A partir des données de numérisation et des marqueurs informatifs, un logiciel détermine automatiquement l'axe de l'élément de cicatrisation, par construction géométrique, par exemple à partir de l'identification du centre 15 du capuchon et de la direction perpendiculaire à la surface terminale 14 passant par ce centre 15, puis son orientation et l'espace qu'il occupe, en utilisant les marqueurs. Il peut ainsi notamment déterminer automatiquement l'axe de l'implant, sans avoir à le visualiser directement. En effet, l'élément de cicatrisation est avantageusement aligné avec l'implant, son axe étant ainsi confondu avec celui de l'implant.

En effet, il est possible de déduire des marqueurs informatifs l'orientation de l'axe du dispositif de connexion avec l'implant, par exemple le dispositif de connexion 3 de l'embase de pilier avec l'implant : cela permet d'en déduire automatiquement le positionnement du dispositif de connexion de l'implant, sans avoir à le visualiser directement, à partir de la connaissance de l'embase de pilier.

Enfin, dans le cas où des éléments de cicatrisation de hauteur différente existent, il reste à déterminer cette hauteur, pour positionner parfaitement l'implant invisible. Une première approche peut consister à former des éléments de cicatrisation de couleur différente pour des hauteurs différentes. Une seconde approche consiste à prévoir et à identifier au moins un marqueur informatif sur l'élément de cicatrisation pour indiquer cette hauteur.

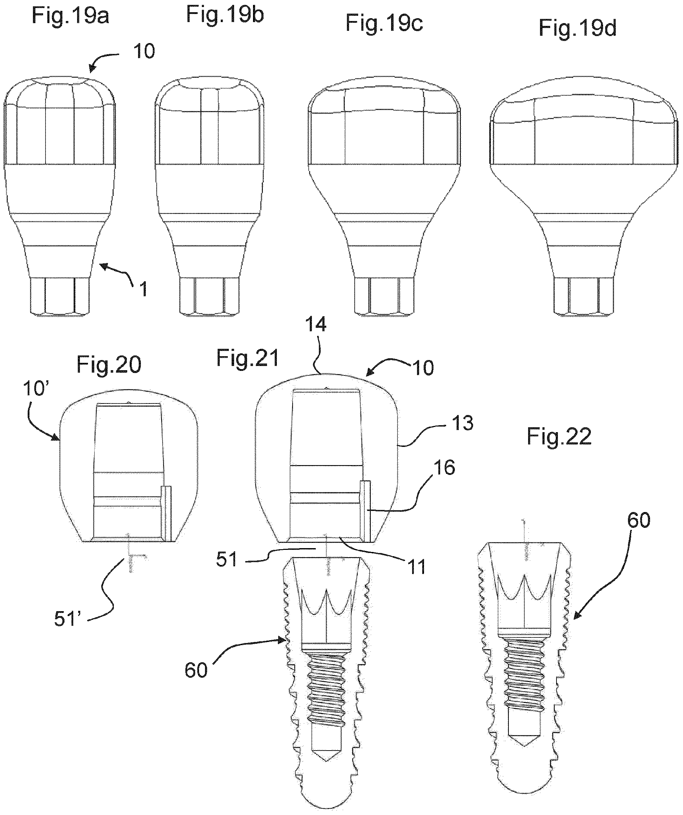

En variante ou complément de réalisation, un opérateur saisit par une interface homme machine la référence de l'élément de cicatrisation, ce qui permet au logiciel de retrouver certaines caractéristiques de cet élément de cicatrisation, en confirmation ou en complément de celles obtenues par les marqueurs, comme sa hauteur, son centre et/ou axe, dans une bibliothèque présente sous forme de base de données stockée dans une mémoire électronique qu'il peut consulter. La figure 20 illustre à titre d'exemple un capuchon 10' virtuel mémorisé dans la bibliothèque associée au logiciel de restauration. Un repère dans l'espace 51' est associé au capuchon, permettant son positionnement dans l'espace. En variante, le logiciel peut reconnaître automatiquement l'élément de cicatrisation à partir de ses marqueurs informatifs, voire de ses autres caractéristiques géométriques, sans saisie manuelle de sa référence. Un opérateur peut assister un logiciel au bon positionnement du repère 51 du capuchon réel, c'est-à-dire à la reconnaissance de son positionnement réel, en saisissant sur une image obtenue par l'étape de numérisation mentionnée ci-dessus et présentée à l'opérateur sur un écran d'une interface homme machine un ou plusieurs points de la surface émergente.

A partir des données numérisées, et éventuellement de l'aide du ou des points de la surface de l'élément de cicatrisation manuellement renseignés par un opérateur, le logiciel sait donc associer l'élément de cicatrisation virtuel issu de sa bibliothèque à l'environnement buccal numérisé, en remplacement de l'élément de cicatrisation réel, pour obtenir une reproduction numérique plus parfaite. Comme représenté sur la figure 21, le repère réel 51 du capuchon 10 réel est ainsi déterminé automatiquement par le logiciel. Il est possible de positionner parfaitement l'élément de cicatrisation virtuel sur l'empreinte numérique, automatiquement ou éventuellement par l'intermédiaire d'une intervention d'un opérateur sur une interface homme machine permettant de visualiser l'empreinte buccale et l'élément de cicatrisation. Ce positionnement parfait de l'élément de cicatrisation virtuel permet d'en déduire toutes les géométries avoisinantes, à partir des références connues mémorisées dans la base de données associée à l'élément de cicatrisation précis considéré, dont la position de l'implant 60 et la géométrie de la gencive cicatrisée sans présence du capuchon 10 ni de l'embase de pilier, comme représenté par la figure 22. En variante, les marqueurs utilisés suffisent à fournir les données nécessaires au bon positionnement du capuchon sans recours à une bibliothèque.

Dans tous les cas, lorsque le logiciel de restauration a repositionné avec exactitude le positionnement de l'implant caché, il déduit de ces connaissances la géométrie finale du pilier de restauration à fabriquer, qui doit être fixé à l'implant et occuper tout le volume gingival défini par l'élément de cicatrisation, puis la géométrie de la prothèse dentaire destinée à se fixer sur ce pilier, de manière connue.

En remarque, ce procédé de restauration peut se faire totalement numériquement, donc virtuellement, de manière totalement ou partiellement automatisée, ou comprendre des phases de construction d'une maquette en plastique ou plâtre. Dans ce dernier cas, une empreinte physique, par exemple en silicone, peut être réalisée, un plâtre peut être coulé dans l'empreinte pour créer le maître modèle, c'est à dire une réplique de l'arcade dentaire à restaurer, qui est ensuite scannée en laboratoire pour reconstruire une image numérique.

Comme cela ressort de la description ci-dessus, la dernière phase du procédé de restauration repose donc sur un dispositif de restauration, qui comprend une unité centrale de traitement et de commande, comprenant ici au moins un microprocesseur, lié à une mémoire électronique, sur lequel est exécuté un logiciel permettant la mise en oeuvre de tout ou partie des étapes du procédé de restauration décrit ci-dessus. Cette unité centrale est liée par un dispositif de communication à un module d'obtention de données numériques représentant tout ou partie d'une dentition d'un patient, qui peut consister en un appareil comme un scanner buccal. Elle est aussi liée à une interface homme machine, comprenant par exemple un écran et/ou un clavier, pour permettre les échanges avec un opérateur, comme explicité ci-dessus. L'unité centrale réalise ensuite tous les traitements nécessaires, calculs et autres, par un moyen logiciel. Elle est enfin apte à générer et transmettre des commandes de fabrication à un dispositif de fabrication d'un pilier de restauration et/ou de prothèse. Elle peut de plus être liée par un second dispositif de communication à un dispositif de fabrication comme une machine outil.

L'invention porte aussi sur un élément de cicatrisation apte à une connexion avec un implant dentaire 60, comprenant une surface latérale 13 destinée à une intégration au sein d'une gencive pour mettre en forme la gencive lors de sa cicatrisation, et une surface terminale 14, caractérisé en ce qu'une partie de la surface latérale 13 et de la surface terminale 14 forment une surface émergente, qui est asymétrique par rapport à au moins un plan médian perpendiculaire à la surface émergente et passant par le centre de la surface émergente ou comprenant un axe 18 central de l'élément de cicatrisation et en ce que la surface émergente comprend au moins deux marqueurs informatifs 70 permettant l'identification d'au moins deux caractéristiques dudit élément de cicatrisation et/ou d'une embase de pilier 1 et/ou dudit implant dentaire 60 sur lequel il serait connecté, et en ce que sa surface terminale14) et/ou sa surface émergente présente :
- une surface sans aspérité en dehors des marqueurs informatifs 70 ; et/ou
- une surface sans ouverture débouchante.

L'élément de cicatrisation peut être unitaire, adapté à une fixation directe sur un implant dentaire 60 sans utilisation d'une embase de pilier.

Une section transverse à la surface latérale 13 de l'élément de cicatrisation ou une projection sur un plan parallèle à la surface terminale 14 de la surface émergente de l'élément de cicatrisation peut présenter :
- une forme sensiblement trapézoïdale ou une forme sensiblement polygonale, ou triangulaire, ou carrée, ou rectangulaire, ou ovoïde, ou une forme sensiblement polygonale avec des angles arrondis ; et/ou
- une partie destinée à un positionnement orienté vers l'extérieur de la bouche de plus grande dimension qu'une partie destinée à un positionnement orienté vers l'intérieur.

La surface terminale 14 et/ou la surface émergente peut présenter :
- une surface non plane, courbée ; et/ou
- une surface convexe.

Lesdites caractéristiques identifiées par les marqueurs informatifs 70 peuvent comprendre un ou plusieurs éléments parmi :
- la hauteur de l'élément de cicatrisation,
- la forme de l'élément de cicatrisation, notamment la forme et les dimensions de la section transverse de sa surface latérale 13 ou de la projection sur un plan parallèle de la surface émergente,
- les dimensions de la partie de liaison de l'élément de cicatrisation 10 avec une embase de pilier 1, et indirectement d'un l'implant dentaire 60,
- l'orientation dudit implant dentaire 60, par l'intermédiaire de l'orientation de l'élément de cicatrisation 10.

Lesdits au moins deux marqueurs informatifs 70 peuvent comprendre un premier type de marquage informatif d'une première caractéristique de l'élément de cicatrisation 10 et un deuxième type de marquage informatif d'une deuxième caractéristique distincte de l'élément de cicatrisation 10.

Chaque marqueur informatif peut appartenir à l'un des types suivants :
- un marqueur informatif négatif, notamment en creux dans l'une des surfaces de l'élément de cicatrisation,
- un marqueur informatif positif, notamment en relief sur l'une des surfaces de l'élément de cicatrisation,
- un marqueur informatif de forme particulière et identifiable formée dans l'une des surfaces de l'élément de cicatrisation, notamment de forme polygonale ou de ligne,
- un marqueur informatif constitué par une valeur numérique lisible ou par un code d'identification lisible tel qu'un code barres ou un code datamatrix,
- une puce RFID.

L'invention porte aussi sur une série d'éléments de cicatrisation, caractérisée en ce qu'elle comprend au moins deux éléments de cicatrisation tel que décrit précédemment présentant des formes différentes, ou en ce qu'elle comprend au moins trois éléments de cicatrisation tel que décrit précédemment de formes différentes.

Lesdits au moins deux ou au moins trois éléments de cicatrisation peuvent présenter des hauteurs différentes entre eux et/ou présenter des sections transverses de leurs surfaces latérales 13 ou des projections sur un plan parallèle de leurs surfaces terminales 14 différentes entre elles.

L'invention porte aussi sur un procédé de fabrication d'un pilier de restauration dentaire et/ou d'une prothèse, destiné à être fixé sur un implant dentaire sur une première extrémité, caractérisé en ce qu'il comprend les étapes suivantes :
- réalisation d'une empreinte physique ou numérique de l'espace buccal comprenant un élément de cicatrisation connecté à un implant dentaire 60, l'élément de cicatrisation comprenant une surface latérale 13 destinée à une intégration au sein d'une gencive pour mettre en forme la gencive lors de sa cicatrisation, et une surface terminale 14, une partie de la surface latérale 13 et de la surface terminale 14 formant une surface émergente, qui est asymétrique par rapport à au moins un plan médian perpendiculaire à la surface émergente et passant par le centre de la surface émergente ou comprenant un axe 18 central de l'élément de cicatrisation et la surface émergente comprenant au moins deux marqueurs informatifs 70 permettant l'identification d'au moins deux caractéristiques dudit élément de cicatrisation et/ou d'une embase de pilier 1 et/ou dudit implant dentaire 60 sur lequel il est connecté,
- détermination automatique et/ou à partir de données saisies sur une interface homme machine par un opérateur du positionnement de l'implant dentaire et de l'espace buccal, à partir de ladite empreinte et des marqueurs informatifs de l'élément de cicatrisation 10.

Le procédé peut comprendre une étape de détermination automatique de l'axe de l'élément de cicatrisation, à partir de l'identification du centre 15 de l'élément de cicatrisation et de la direction perpendiculaire à la surface terminale 14 passant par ce centre 15, une étape de détermination automatique de l'axe de l'implant, confondu avec l'axe de l'élément de cicatrisation, et une étape de détermination de l'orientation de l'élément de cicatrisation à partir des marqueurs.

Le procédé peut comprendre une étape de détermination de la hauteur de l'élément de cicatrisation, à partir d'éléments de cicatrisation de couleur différente pour des hauteurs différentes ou en identifiant au moins un marqueur informatif qui indique cette hauteur sur l'élément de cicatrisation.

Le procédé peut comprendre une étape de saisie par une interface homme machine de la référence de l'élément de cicatrisation, puis une étape consistant à retrouver des caractéristiques de cet élément de cicatrisation, en confirmation ou en complément de celles obtenues par les marqueurs, parmi sa hauteur, son centre et/ou axe, dans une bibliothèque présente sous forme de base de données stockée dans une mémoire électronique.

Le procédé peut comprendre une étape de saisie d'un ou plusieurs points de la surface émergente par une interface homme machine sur une image obtenue par l'étape de réalisation d'une empreinte numérique de l'espace buccal.

Le procédé peut comprendre une étape de déduction de la géométrie finale du pilier de restauration à fabriquer, qui doit être fixé à l'implant dentaire de sorte à occuper tout le volume gingival défini par l'élément de cicatrisation, et de détermination de la géométrie de la prothèse dentaire destinée à se fixer sur ce pilier de restauration.

## Revendications

1. Elément de cicatrisation unitaire, adapté à une fixation directe sur un implant dentaire (60) sans utilisation d'une embase de pilier, comprenant une surface latérale (13) destinée à une intégration au sein d'une gencive pour mettre en forme, selon une forme anatomique, la gencive lors de sa cicatrisation, et une surface terminale (14), **caractérisé en ce qu'**une partie de la surface latérale (13) et de la surface terminale (14) forment une surface émergente, qui est asymétrique par rapport à au moins un plan médian perpendiculaire à la surface émergente et passant par le centre de la surface émergente ou comprenant un axe (18) central de l'élément de cicatrisation et **en ce que** la surface émergente comprend au moins deux marqueurs informatifs (70) permettant l'identification d'au moins deux caractéristiques dudit élément de cicatrisation et/ou dudit implant dentaire (60) sur lequel il serait connecté, lesdits au moins deux marqueurs informatifs (70) comprenant un premier type de marquage informatif d'une première caractéristique de l'élément de cicatrisation et un deuxième type de marquage informatif (70) d'une deuxième caractéristique distincte de l'élément de cicatrisation.

2. Elément de cicatrisation selon la revendication précédente, **caractérisé en ce que** lesdites caractéristiques identifiées par les marqueurs informatifs (70) comprennent un ou plusieurs éléments parmi :
- la hauteur de l'élément de cicatrisation,
- l'orientation de l'élément de cicatrisation (10), et l'orientation dudit implant dentaire (60) par l'intermédiaire de l'orientation de l'élément de cicatrisation (10).

3. Elément de cicatrisation selon l'une des revendications précédentes, caractérisé en ce lesdites caractéristiques identifiées par les marqueurs informatifs (70) comprennent un ou plusieurs éléments parmi :
- la forme de l'élément de cicatrisation, notamment la forme et les dimensions de la section transverse de sa surface latérale (13) ou de la projection sur un plan parallèle de la surface émergente,
- les dimensions de la partie de liaison de l'élément de cicatrisation (10) avec un l'implant dentaire (60).

4. Elément de cicatrisation selon l'une des revendications précédentes, **caractérisé en ce qu'**une section transverse à la surface latérale (13) de l'élément de cicatrisation ou une projection sur un plan parallèle à la surface terminale (14) de la surface émergente de l'élément de cicatrisation présente une forme sensiblement trapézoïdale ou une forme sensiblement polygonale, ou triangulaire, ou carrée, ou rectangulaire, ou ovoïde, ou une forme sensiblement polygonale avec des angles arrondis.

5. Elément de cicatrisation selon l'une des revendications précédentes, **caractérisé en ce qu'**une section transverse à la surface latérale (13) de l'élément de cicatrisation ou une projection sur un plan parallèle à la surface terminale (14) de la surface émergente de l'élément de cicatrisation présente une partie destinée à un positionnement orienté vers l'extérieur de la bouche de plus grande dimension qu'une partie destinée à un positionnement orienté vers l'intérieur.

6. Elément de cicatrisation selon l'une quelconque des revendications 1 à 2, **caractérisé en ce que** sa surface terminale (14) et/ou sa surface émergente présente :
- une surface non plane, courbée ; et/ou
- une surface convexe ; et/ou
- une surface sans aspérité en dehors des marqueurs informatifs ; et/ou
- une surface sans ouverture débouchante.

7. Elément de cicatrisation selon l'une quelconque des revendications précédentes, **caractérisé en ce que** chaque marqueur informatif appartient à l'un des types suivants :
- un marqueur informatif négatif, notamment en creux dans l'une des surfaces de l'élément de cicatrisation,
- un marqueur informatif positif, notamment en relief sur l'une des surfaces de l'élément de cicatrisation,
- un marqueur informatif de forme particulière et identifiable formée dans l'une des surfaces de l'élément de cicatrisation, notamment de forme polygonale ou de ligne,
- un marqueur informatif constitué par une valeur numérique lisible ou par un code d'identification lisible tel qu'un code barres ou un code datamatrix,
- une puce RFID.

8. Série d'éléments de cicatrisation, **caractérisée en ce qu'**elle comprend au moins deux éléments de cicatrisation selon l'une des revendications précédentes présentant des formes différentes, ou **en ce qu'**elle comprend au moins trois éléments de cicatrisation selon l'une des revendications précédentes de formes différentes.

9. Série d'éléments de cicatrisation selon la revendication précédente, **caractérisée en ce que** lesdits au moins deux ou au moins trois éléments de cicatrisation présentent des hauteurs différentes entre eux et/ou présentent des sections transverses de leurs surfaces latérales (13) ou des projections sur un plan parallèle de leurs surfaces terminales (14) différentes entre elles.

10. Procédé de fabrication d'un pilier de restauration dentaire et/ou d'une prothèse, destiné à être fixé sur un implant dentaire sur une première extrémité, **caractérisé en ce qu'**il comprend les étapes suivantes :
- réalisation d'une empreinte physique ou numérique de l'espace buccal comprenant un élément de cicatrisation unitaire directement connecté à un implant dentaire (60) sans utilisation d'une embase de pilier, l'élément de cicatrisation comprenant une surface latérale (13) destinée à une intégration au sein d'une gencive pour mettre en forme la gencive lors de sa cicatrisation, selon une forme anatomique, et une surface terminale (14), une partie de la surface latérale (13) et de la surface terminale (14) formant une surface émergente, qui est asymétrique par rapport à au moins un plan médian perpendiculaire à la surface émergente et passant par le centre de la surface émergente ou comprenant un axe (18) central de l'élément de cicatrisation et la surface émergente comprenant au moins deux marqueurs informatifs (70) permettant l'identification d'au moins deux caractéristiques dudit élément de cicatrisation et/ou dudit implant dentaire (60) sur lequel il est connecté,
- détermination automatique et/ou à partir de données saisies sur une interface homme machine par un opérateur du positionnement de l'implant dentaire et de l'espace buccal, à partir de ladite empreinte et des marqueurs informatifs de l'élément de cicatrisation (10).

11. Procédé de fabrication d'un pilier de restauration dentaire et/ou d'une prothèse selon la revendication précédente, **caractérisé en ce qu'**il comprend une étape de détermination automatique de l'axe de l'élément de cicatrisation, à partir de l'identification du centre (15) de l'élément de cicatrisation et de la direction perpendiculaire à la surface terminale (14) passant par ce centre (15), une étape de détermination automatique de l'axe de l'implant, confondu avec l'axe de l'élément de cicatrisation, et une étape de détermination de l'orientation de l'élément de cicatrisation à partir des marqueurs.

12. Procédé de fabrication d'un pilier de restauration dentaire et/ou d'une prothèse selon la revendication 10 ou 11, **caractérisé en ce qu'**il comprend une étape de détermination de la hauteur de l'élément de cicatrisation, à partir d'éléments de cicatrisation de couleur différente pour des hauteurs différentes ou en identifiant au moins un marqueur informatif qui indique cette hauteur sur l'élément de cicatrisation.

13. Procédé de fabrication d'un pilier de restauration dentaire et/ou d'une prothèse selon la revendication 11 ou 12, **caractérisé en ce qu'**il comprend une étape de saisie par une interface homme machine de la référence de l'élément de cicatrisation, puis une étape consistant à retrouver des caractéristiques de cet élément de cicatrisation, en confirmation ou en complément de celles obtenues par les marqueurs, parmi sa hauteur, son centre et/ou axe, dans une bibliothèque présente sous forme de base de données stockée dans une mémoire électronique.

14. Procédé de fabrication d'un pilier de restauration dentaire et/ou d'une prothèse selon l'une des revendication 10 à 13, **caractérisé en ce qu'**il comprend une étape de saisie d'un ou plusieurs points de la surface émergente par une interface homme machine sur une image obtenue par l'étape de réalisation d'une empreinte numérique de l'espace buccal.

15. Procédé de fabrication d'un pilier de restauration dentaire et d'une prothèse selon l'une des revendications 10 à 14, **caractérisé en ce qu'**il comprend une étape de déduction de la géométrie finale du pilier de restauration à fabriquer, qui doit être fixé à l'implant dentaire de sorte à occuper tout le volume gingival défini par l'élément de cicatrisation, et de détermination de la géométrie de la prothèse dentaire destinée à se fixer sur ce pilier de restauration.
